(19) Europäisches Patentamt
European Patent Office
Office européen des brevets

(11) Numéro de publication : **0 422 968 B1**

(12) # FASCICULE DE BREVET EUROPEEN

(45) Date de publication du fascicule du brevet : **05.01.94 Bulletin 94/01**

(51) Int. Cl.$^5$ : **C07C 29/14,** C07C 33/02

(21) Numéro de dépôt : **90402402.3**

(22) Date de dépôt : **30.08.90**

(54) **Hydrogénation du citral.**

(30) Priorité : **13.10.89 FR 8913518**

(43) Date de publication de la demande : **17.04.91 Bulletin 91/16**

(45) Mention de la délivrance du brevet : **05.01.94 Bulletin 94/01**

(84) Etats contractants désignés : **CH DE ES GB IT LI NL**

(56) Documents cités :
**EP-A- 0 071 787**
**FR-A- 2 208 879**
**GB-A- 2 052 294**
**CHEMICAL ABSTRACTS OF JAPAN, vol. 83, 1975, page 393, colonne 1, résumé no. 192552q, Columbus, Ohio, US; & JP-A-7588010**

(73) Titulaire : **INSTITUT FRANCAIS DU PETROLE**
**4, Avenue de Bois Préau**
**F-92502 Rueil-Malmaison (FR)**

(72) Inventeur : **Didillon, Blaise**
**15, rue du Bâtonnier Jacquier**
**F-69007 Lyon (FR)**
Inventeur : **Candy, Jean-Pierre**
**4, chemin de la Mascotte**
**F-69300 Caluire (FR)**
Inventeur : **Basset, Jean-Marie**
**21, Petite Rue de la Dova**
**F-69100 Villeurbanne (FR)**
Inventeur : **Bournonville, Jean-Paul**
**43, rue des Groues Vauréal**
**F-95000 Cergy Pontoise (FR)**

(74) Mandataire : **Andreeff, François et al**
**INSTITUT FRANCAIS DU PETROLE 4, avenue de Bois-Préau**
**F-92502 Rueil-Malmaison (FR)**

**EP 0 422 968 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention concerne un procédé de production d'alcools insaturés dioléfiniques (et notamment le géraniol et le nérol) par hydrogénation sélective de citral.

Le citral est un aldéhyde contenant deux liaisons carbone-carbone oléfiniques et répond à la formule brute suivante :

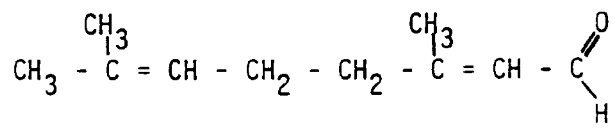

3,7 diméthyl 2,6 octadiène - al ou citral ($C_{10}H_{16}O$).

L'hydrogénation sélective de la fonction aldéhydique conduit à la formation d'alcools insaturés isomères tels que le géraniol et le nérol ayant tous les deux la formule développée brute suivante :

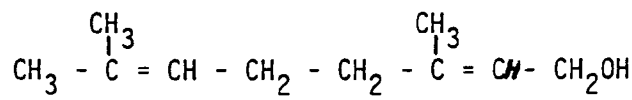

3,7 diméthyl 2,6 octadiène - 1 ol (nérol ou géraniol).

Ces produits sont utilisés notamment dans l'industrie de la parfumerie.

La grande difficulté de cette réaction est l'obtention de conversion élevée, si possible totale, avec la meilleure sélectivité possible, c'est-à-dire la plus proche possible de 100 % , en alcools dioléfiniques. En effet les alcools dioléfiniques isomères, géraniol et nérol, peuvent subir à leur tour une hydrogénation partielle ou totale des doubles liaisons oléfiniques qui conduit aux deux produits suivants :
d'une part le

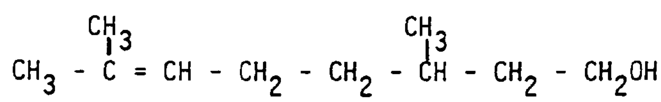

3,7 diméthyl - 6 octène - 1 ol ou citronellol dans le cas de l'hydrogénation de la double liaison située en $\alpha$ de la fonction alcool
d'autre part le

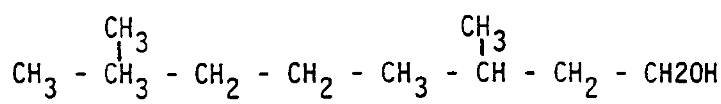

3,7 diméthyl octanol 1

quand toutes les liaisons insaturées carbone-carbone et carbone-oxygène sont hydrogénées.

Par ailleurs, la double liaison oléfinique conjuguée avec la fonction aldéhyde peut également être hydrogénée pour donner un composé répondant à la formule brute suivante :

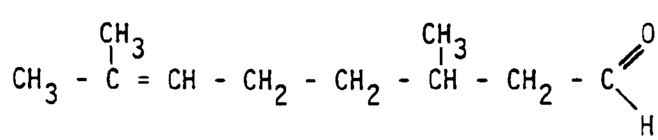

3,7-diméthyl 7-octénal ou citronellal qui après l'hydrogénation de la double liaison oléfinique conduit au 3,7 diméthyl octanal.

Les métaux du groupe VIII sont connus comme catalyseurs de la transformation du citral sous pression d'hydrogène et en phase liquide, c'est-à-dire en présence d'un solvant. Ainsi l'hydrogénation sélective de citral en citronellal a été revendiquée en présence de catalyseurs à base de palladium (EP-A-008741 et FR-B-2405232) de même que l'hydrogénation du citral en 3,7 diméthyl octanal (DE 2832699). Les chlorures de cobalt et de nickel, associés en solution à du cyanure de potassium et une amine aliphatique, permettent également d'obtenir sous pression d'hydrogène des rendements très élevés en citronellal à partir de citral (SU958.408).

La transformation du citral en citronellol fait l'objet d'un certain nombre de publications. Ainsi un rendement de 93 % de citronellol est obtenu sous pression d'hydrogène à partir de citral en présence d'un catalyseur contenant du palladium associé à de l'iridium, de l'osmium, du platine, du rhodium ou du ruthénium et avec une amine aliphatique (DE 293250). Le nickel, le rhodium, le platine ou le palladium, sous forme massique, permettent d'atteindre des sélectivités de production en citronellol à partir de citral supérieures à 90 % (Kinet. Catal 21(3), 670-5). Le nickel déposé sur oxyde de chrome permet, en milieu alcoolique et en présence de carbonate de sodium, de réaliser des rendements de 95 % en citronellol à partir de citral (Reaction Kinetics and Catalysis Letters, 16(4), 339-43).

En ce qui concerne l'hydrogénation sélective du citral en géraniol et nérol en phase liquide ($CH_3OH$), l'obtention de sélectivités élevées (93-95 %) à conversion limitée a été revendiquée (US-4100180) en présence d'oxyde de platine $PtO_2$ promu par $FeSO_4$ et Zn $(CH_3COO)_2$. Le catalyseur peut être réutilisé à condition d'ajouter les composés du fer et du zinc avec chaque charge de citral. De même un catalyseur à base de platine supporté sur charbon permet d'obtenir des sélectivités en géraniol et nérol élevées (>95 %) pour des conversions du citral comprises en 92 et 97 % (US 4073813). Enfin deux brevets de la société BASF AG revendiquent l'utilisation de catalyseurs, l'un contenant 5 % poids de Pd, Ir, Os, Rh, Ru ou Pt supporté sur charbon en présence d'une amine aliphatique (US-4455442), et l'autre 5 % de ruthénium supporté sur charbon promu par du fer en présence également d'une amine aliphatique (US-4465787). Ces catalyseurs ont des activités et des sélectivités élevées mais nécessitent des ajouts de composés chimiques bien définis.

Il a été découvert dans la présente invention qu'il est possible de réaliser l'hydrogénation sélective du citral ou 3,7 diméthyl 2-octadiénal en géraniol et nérol avec une obtention réduite de citronellal. On opère dans un réacteur continu ou discontinu en présence d'hydrogène sous une pression totale comprise entre 10 et 100 bars (1 et 10 Mégapascals) et de préférence entre 20 et 80 bars (2 et 8 Mégapascals), ou mieux au-dessus de 30 bars (3 Mégapascals), bien que l'on puisse opérer sans inconvénient, par exemple, jusqu'à 300 bars (30 Mégapascals), à une température comprise entre 0 et 100 degrés Celsius et de préférence entre 30 et 80 degrés Celsius en présence d'un catalyseur métallique supporté renfermant (a) au moins un métal du groupe VIII choisi parmi l'iridium, le platine, le rhodium et le ruthénium (le rhodium et le ruthénium étant les métaux préférés) et dont le pourcentage pondéral est choisi entre 0,1 et 10 % et de préférence entre 0,5 et 5 % et (b) au moins un élément additionnel métallique choisi dans le groupe IV.A constitué par l'étain, le germanium et le plomb dont le pourcentage pondéral est choisi entre 0,01 et 10 % et de préférence entre 0,1 et 5 %. Le rapport molaire élément métallique du groupe IV sur métal du groupe VIII est avantageusement compris entre 0,8 et 3 et de préférence entre 0,9 et 2,6. On peut avantageusement dans certains cas utiliser à la fois deux des métaux du groupe ci-dessus IV-A ou même les trois métaux du groupe ; le support, quand il en est utilisé un, peut être choisi dans le groupe constitué par la silice, les différents types d'alumine, les silices alumines, les aluminates des éléments des groupes $I_A$, $II_A$ ou $II_B$ de la classification périodique des éléments comme par exemple les aluminates de Ca, Mg, Ba, Zn, Na, K, Cd et les aluminates mixtes, et le charbon, et de préférence dans le groupe constitué par la silice et les aluminates de métaux alcalins et/ou alcalino-terreux et/ou de zinc et/ou cadmium et les aluminates mixtes, et de manière encore plus préférée un support à base de silice.

Le catalyseur peut être préparé par différentes procédures d'imprégnation du support et l'invention n'est pas limitée à une procédure déterminée. L'opération d'imprégnation consiste, par exemple, à mettre en contact le support préformé et une solution aqueuse ou organique d'un composé du métal ou des métaux choisi(s), le volume de solution étant de préférence en excès par rapport au volume de rétention du support ou égal à ce volume. On peut introduire le métal du groupe VIII et le métal additionnel simultanément ou successivement. Après avoir laissé le contact entre le support et la solution pendant plusieurs heures, le support imprégné est filtré, lavé à l'eau distillée, séché et calciné sous air habituellement entre 110 et 600°C et de préférence entre 110 et 500°C. Avant utilisation, on réduit le catalyseur sous hydrogène habituellement entre 50 et 600°C et de préférence entre 90 et 500°C, cette réduction pouvant être effectuée aussitôt après calcination, ou plus tard chez l'utilisateur.

L'élément choisi dans le groupe constitué par l'étain, le germanium et le plomb peut être introduit en solution aqueuse ou en solution hydrocarbonée selon la nature du précurseur utilisé.

D'une manière préférée le catalyseur est obtenu par imprégnation du support, à l'aide d'une solution aqueuse ou organique d'au moins un composé de métal du groupe VIII, le volume de solution étant de préférence en excès par rapport au volume de rétention du support ou égal à ce volume. Le support imprégné est

ensuite filtré, éventuellement lavé à l'eau distillée puis séché et calciné sous air habituellement entre environ 110°C et environ 600°C, et de préférence entre environ 110°C et environ 500°C, puis ensuite réduit sous hydrogène à une température habituellement comprise entre environ 200°C et environ 600°C et de préférence entre environ 300°C et environ 500°C ; le produit obtenu est alors imprégné par une solution aqueuse ou organique d'un composé de germanium, d'étain et/ou de plomb ; d'une manière particulièrement avantageuse, on utilise une solution d'au moins un hydrocarbyl-germanium, un hydrocarbyl-étain ou un hydrocarbyl-plomb dans un hydrocarbure saturé, selon la technologie décrite dans le brevet US-A-4.548.918 de la demanderesse.

Parmi les solvants organiques utilisables dans le cadre de l'invention on peut citer à titre d'exemples non limitatifs les hydrocarbures, les hydrocarbures halogénés, les cétones et les éthers. L'emploi d'un solvant n'est pas indispensable lorsque le composé de germanium, d'étain et/ou de plomb est lui-même liquide comme cela est par exemple le cas pour le tétrabutylétain.

Après avoir laissé le contact entre le support imprégné du métal du groupe VIII et la solution contenant au moins un composé de germanium, étain ou plomb pendant plusieurs heures, le produit est filtré, éventuellement lavé à l'aide du solvant employé pour déposer le germanium, l'étain et/ou le plomb, séché et éventuellement calciné sous air habituellement entre environ 110°C et environ 600°C, de préférence entre environ 110°C et 500°C. Avant utilisation on réduit le catalyseur sous hydrogène habituellement entre environ 50 et environ 600°C et de préférence entre environ 90°C et environ 500°C, cette réduction pouvant être effectuée aussitôt après la calcination, ou plus tard chez l'utilisateur.

Une autre méthode consiste à malaxer la poudre humide de support avec les précurseurs du catalyseur et à mettre ensuite en forme et sécher.

Les exemples des précurseurs métalliques utilisables dans la préparation du catalyseur sont les suivants :

Pour le métal du groupe VIII, on peut utiliser des composés tels que les chlorures les nitrates, les composés halogéno-amminés, les composés amminés, les sels d'acides organiques solubles dans le solvant d'imprégnation.

On peut aussi utiliser des composés organométalliques d'un métal du groupe VIII en solution dans un solvant organique, par exemple un hydrocarbure. Comme exemples d'hydrocarbures on peut citer les hydrocarbures paraffiniques saturés dont la chaîne hydrocarbonée renferme de 6 à 12 atomes de carbone par molécule, les hydrocarbures naphténiques qui renferment de 6 à 12 atomes de carbone dans leur molécule ou encore les hydrocarbures aromatiques renfermant de 6 à 11 atomes dans leur molécule. A titre d'exemples de composés organométalliques de métal du groupe VIII on peut citer : les composés carbonyles, halogénocarbonyles et les acétylacétonates sans que cette liste soit limitative.

L'élément choisi dans le groupe constitué par l'étain, le germanium et le plomb peut être introduit de préférence sous la forme d'au moins un composé organique choisi dans le groupe formé par les complexes, en particulier les complexes polycétoniques, des métaux du groupe IV.A et les hydrocarbylmétaux tels que les alkyles, les cycloalkyles, les aryles, les alkylaryles et les arylalkyles métaux.

L'introduction du métal IV.A est avantageusement effectuée à l'aide d'une solution dans un solvant organique du composé organométallique dudit métal IV.A. On peut également employer des composés organohalogénés des métaux IV.A. Comme composés de métaux IV.A on citera en particulier le tétrabutyl-étain, le tétraméthyl-étain, le tétrapropyl-étain, le tétrapropyl-germanium, le tétraéthyl-plomb, le diphényl-étain, le diphényl-germanium, le tétraphényl-plomb.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer le n-heptane, le méthylcyclohexane, le toluène et le chloroforme. On peut utiliser des mélanges des solvants définis ci-dessus.

L'élément choisi dans le groupe constitué de l'étain, du germanium et du plomb peut aussi être introduit par l'intermédiaire de composés tels que les chlorures, les bromures et le nitrate d'étain, les halogénures, nitrate et acétate de plomb, le chlorure et l'oxalate de germanium en solution aqueuse ou organique.

Le support peut être de nature variée, comme déjà mentionné plus haut, mais il est de préférence à base de silice. Un support particulièrement adapté possède des caractéristiques spécifiques telles qu'une surface spécifique,, déterminée par la méthode B.E.T., comprise entre 10 et 500 mètres carrés par gramme et de préférence comprise entre 50 et 500 mètres carrés par gramme et un volume poreux total de 0,2 à 1,3 $cm^3$/g de support.

Une fois les métaux fixés sur le support, le catalyseur subit avantageusement un traitement d'activation sous hydrogène à haute température, par exemple 50-600°C, afin d'obtenir une phase métallique active. La procédure de ce traitement sous hydrogène consiste par exemple en une montée lente de la température sous courant d'hydrogène jusqu'à la température maximale de réduction, comprise par exemple entre 50 et 600°C et de préférence entre 90 et 500°C, suivie d'un maintien pendant par exemple 1 à 6 heures à cette température.

Les exemples suivants, non limitatifs, illustrent l'invention.

EXEMPLE 1.

La préparation du catalyseur se fait en deux étapes :

- tout d'abord fixation du rhodium par imprégnation de chlorure de rhodium chloropentammine en solution ammoniacale sur une silice dont la surface spécifique est égale à 280 $m^2$ par gramme et le volume poreux total est égal à 80 $cm^3$ pour 100 grammes, suivie d'une filtration, d'un séchage à 110°C, d'une calcination sous air à 450°C et d'une réduction sous hydrogène à 450°C,
- le catalyseur est ensuite chargé dans un réacteur de type guignard contenant un solvant organique, le normal hexane. Le tétrabutyl-étain est alors injecté dans le solvant. Le réacteur est ensuite fermé. La pression d'hydrogène est augmentée jusqu'à 40 bars (4 Mégapascals) et la température élevée jusqu'à 96°C. Ces conditions sont maintenues pendant 20 minutes sous agitation.

Le catalyseur monométallique de départ (c'est-à-dire sans ajout d'étain) contient 1,3 % poids de rhodium.

Une série de catalyseurs contenant différentes concentrations en étain a été préparée selon la méthode décrite ci-dessus. La composition de ces catalyseurs est donnée dans le tableau 1.

TABLEAU 1

| Catalyseur | % Rh (pds) | % Sn (pds) | Sn/Rh | |
|---|---|---|---|---|
| | | | molaire | pondéral |
| A | 1,3 | 0,15 | 0,1 | 0,11 |
| B | 1,3 | 0,75 | 0,5 | 0,57 |
| C | 1,3 | 1,5 | 1,0 | 1,15 |
| D | 1,3 | 3,75 | 2,5 | 2,88 |

EXEMPLE 2.

Les catalyseurs de l'exemple 1 sont ensuite testés dans la réaction d'hydrogénation du citral dans les conditions opératoires suivantes :

- température : 65°C
- pression d'hydrogène = 76 bars (7,6 Mégapascals)
- rapport citral/rhodium = 200.

La procédure de test est la suivante :

- après avoir fait réagir le tétrabutyl étain dans les conditions décrites dans l'exemple 1, la pression du réacteur est ramenée jusqu'à la pression atmosphérique et la température abaissée à 25°C centigrade,
- une solution de citral dans le n-hexane est alors injectée dans le réacteur dans une proportion de 200 molécules d'aldéhyde dioléfinique (citral) par atome gramme de rhodium. Après purge à l'hydrogène, la pression d'hydrogène est montée jusqu'à la valeur désirée (ici 76 bars) ainsi que la température (65°C centigrade). Les résultats donnés dans le tableau 2 sont ceux obtenus après 5 heures de fonctionnement.

TABLEAU 2

| Catalyseur | Conversion (% poids) | Sélectivité (% poids) citronellol | citronellal | géraniol nérol | 3,7 DMe octanol |
|---|---|---|---|---|---|
| A | 100,0 | 12,8 | 81,0 | 6,2 | - |
| B | 91,4 | 16,9 | 13,7 | 69,3 | - |
| C | 95,7 | 5,4 | - | 94,6 | - |
| D | 100,0 | 3,5 | - | 96,5 | - |

L'augmentation de la teneur en étain (ou plus exactement du rapport étain/rhodium) permet donc d'améliorer très sensiblement la sélectivité en géraniol et nérol.

EXEMPLE 3.

Après une hydrogénation du citral en présence du catalyseur D, la pression dans le réacteur est abaissée jusqu'à la pression atmosphérique et le réacteur vidé de la phase liquide contenant le solvant et les produits d'hydrogénation. Une nouvelle charge de citral, diluée dans le n-hexane, est alors injectée dans le réacteur, la pression d'hydrogène et la température sont alors ajustées aux-mêmes valeurs que celles de l'exemple 2. Au bout de cinq heures les produits de la réaction sont analysés. L'opération peut alors être répétée à loisir.

Dans le tableau 3 sont rassemblés les résultats de 5 hydrogénations successives avec la même charge de catalyseur.

TABLEAU 3

| Hydrogénation | Conversion (% poids) | Sélectivité (% poids) Citronellol | Citronellal | Nérol géraniol | 37-DMe octanol |
|---|---|---|---|---|---|
| 1ère | 100,0 | 3,5 | - | 96,5 | - |
| 2ème | 100,0 | 3,8 | - | 96,2 | - |
| 3ème | 100,0 | 3,4 | - | 96,6 | - |
| 4ème | 100,0 | 4,1 | - | 95,9 | - |
| 5ème | 100,0 | 3,6 | - | 96,4 | - |

La même charge de catalyseur peut donc être utilisée plusieurs fois de suite sans modification notable de ses propriétés catalytiques.

EXEMPLE 4.

Le catalyseur C est utilisé dans les conditions de l'exemple 2, si ce n'est que la température de réaction est ajustée à 51°C. Au bout de 5 heures de réaction les produits de réaction sont analysés. Les résultats sont les suivants :

- conversion du citral : 100 %
- sélectivité en % poids :
  - citronellol : 5,6
  - citronellal : -
  - géraniol et nérol : 94,4
  - 3,7 diméthyloctanol : -

Les résultats sont voisins de ceux de l'exemple 2.

EXEMPLE 5.

Le catalyseur C est utilisé dans les conditions de l'exemple 2 si ce n'est que la température de réaction est maintenue à 20°C. Dans ce cas pour atteindre une conversion totale du citral il faut attendre 16 heures de réaction. Après analyse des produits de réaction, les résultats peuvent être exprimés de la façon suivante :

- conversion du citral : 100 %
- sélectivité en % poids :
  - 3,7 diméthyl octanol : -
  - citronellal : -
  - citronellol : 8,0
  - géraniol et nérol : 92,0.

La diminution de la température provoque une augmentation du temps de réaction nécessaire pour arriver à une conversion totale moins favorable, quoique très acceptable, à la sélectivité par rapport à l'exemple 2.

EXEMPLE 6.

Le catalyseur C est utilisé selon la procédure opératoire décrite dans l'exemple 2, si ce n'est que la température et la pression d'hydrogène sont respectivement ajustées à 20°C et 20 bars (2 mégapascals).

Après 15 heures de réaction et analyse du milieu réactionnel, les résultats sont alors :

- conversion du citral : 39,4 %
- sélectivité en % poids :
  - 3,7 diméthyl octanol : -
  - citronellol : -
  - citronellal : 5,6
  - géraniol - nérol : 94,4.

La diminution de pression conjuguée à une diminution de température ne permet pas d'améliorer la sélectivité mais diminue l'activité du catalyseur. Il est donc préférable d'opérer simultanément au-dessus de 30°C et au-dessus de 20 bars (2 mégapascals).

EXEMPLE 7.

Le catalyseur A est utilisé dans les conditions de l'exemple 5. Après analyses des produits de réaction, les résultats, au bout de 15 heures, sont les suivants :

- conversion du citral (%) : 100 %
- sélectivité en % poids :
  - 3,7 diméthyl octanol : -
  - citronellol : 18,4
  - citronellal : 81,6
  - nérol et géraniol : -

En comparaison avec l'exemple 2, on voit qu'il est possible d'éviter à la fois la formation de 3,7 diméthyl octanol et de géraniol et nérol en jouant sur la température.

EXEMPLE 8.

Un catalyseur à base de ruthénium en lieu et place du rhodium a également été préparé selon le protocole

suivant :
- fixation du ruthénium par imprégnation de chlorure de ruthénium chloropentammine en solution ammoniacale sur une silice dont la surface spécifique est égale à 280 $m^2$ par gramme et le volume poreux total est égal à 80 $cm^3$ pour 100 grammes, suivies d'une filtration, d'un séchage à 110°C, d'une calcination sous air à 450°C et d'une réduction sous hydrogène à 450°C. Ce catalyseur contient 1 % en poids de ruthénium.

Ce catalyseur est mis en contact avec le tétrabutyl étain dans des conditions identiques à celles de l'exemple 1.

Deux catalyseurs ont ainsi été préparés :

| | Sn/Ru | |
|---|---|---|
| | molaire | poids |
| catalyseur E : 1 % Ru 1,2 % Sn | 1 | 1,2 |
| catalyseur F : 1 % Ru 2,9 Sn | 2,45 | 2,9 |

Ces catalyseurs ont été testés dans les conditions de l'exemple 2 : pression d'hydrogène égale à 76 bars (7,6 mégapascals) et température à 65° centigrades.

Au bout de 7 heures, les résultats sont les suivants

| Catalyseur | Conversion du citral/% poids | Sélectivité % poids | | | |
|---|---|---|---|---|---|
| | | 3,7 DMe octanol | citronellol | citronellal | géraniol nérol |
| E | 99,8 | - | 6,2 | - | 93,8 |
| F | 99,9 | - | 4,0 | - | 96,0 |

<u>EXEMPLE 9.</u>

A partir du catalyseur monométallique à base d'un métal du groupe VIII préparé selon le protocole décrit dans l'exemple 1 pour Rh et l'exemple 8 pour Ru, on se propose de préparer des catalyseurs bimétalliques dans lesquels l'étain est remplacé par le tétrabutylgermane et le tétrabutyl-plomb.

La fixation du deuxième métal du groupe IV (germanium ou plomb) est réalisée dans les mêmes conditions que celles décrites dans l'exemple 1.

Dans le tableau 4 sont rassemblés les compositions des catalyseurs préparés :

| Catalyseur | % métal du groupe VIII | % métal promoteur | IV/VII | |
|---|---|---|---|---|
| | | | molaire | poids |
| G | Rh : 1,1 | Ge : 1,55 | 2,1 | 1,41 |
| H | Rh : 1,1 | Pb : 4,40 | 2,1 | 4 |
| J | Ru : 1,0 | Ge : 1,44 | 2,0 | 1,44 |
| K | Ru : 1,0 | Pb : 4,10 | 2,0 | 4,10 |

Ces catalyseurs ont été utilisés dans la réaction d'hydrogénation du citral dans les mêmes conditions que celles adoptées pour l'exemple 2.

Après 8 heures de réaction, les résultats sont les suivants :

| Catalyseur | Conversion du citral (% poids) | Sélectivité (% poids) | | | |
|---|---|---|---|---|---|
| | | 3,7 diméthyl octanol | citronellol | citronellal | géraniol nérol |
| G | 99,8 | - | 4,1 | - | 95,9 |
| H | 99,1 | - | 5,0 | - | 95,0 |
| J | 99,5 | - | 4,6 | - | 95,4 |
| K | 98,8 | 0,01 | 5,5 | - | 94,5 |

EXEMPLE 10

Un catalyseur monométallique à base de Ru est préparé selon le protocole décrit dans l'exemple 8, mais avec deux supports différents : charbon actif et alumine.

Les caractéristiques des supports sont les suivantes :

- surface spécifique : 200 m$^2$ par gramme pour l'alumine, 850 m$^2$ par gramme pour le charbon.
- volume poreux : 60 cm$^3$ pour 100 grammes pour l'alumine, 80 cm$^3$ pour 100 grammes pour le charbon.

La fixation de l'étain est réalisée dans les mêmes conditions que celles décrites dans l'exemple 1.

Deux catalyseurs ont ainsi été préparés :

| | Sn/Ru | |
|---|---|---|
| | molaire | poids |
| catalyseur L (sur charbon actif)<br>1 % Ru ; 2,9 % Sn | 2,45 | 2,9 |
| catalyseur M (sur alumine)<br>1 % Ru ; 3 % Sn | 2,50 | 3,0 |

Ces catalyseurs sont comparables au catalyseur F (à support silice) de l'exemple 8.

Ils ont été testés dans les conditions de l'exemple 2.

Au bout de 7 heures de réaction, les résultats sont les suivants :

| Catalyseur | Conversion du citral (en poids) | Sélectivité % poids | | | | |
|---|---|---|---|---|---|---|
| | | 3,7 DMe 2,6octadiène | citronellol | citronellal | 3,7 DMe 6 octène | géraniol nérol |
| F | 99,9 | - | 4,0 | - | - | 96,0 |
| L | 90 0 | - | 6,5 | - | - | 93,5 |
| M | 95,0 | 7 | 2,5 | - | 1,5 | 89 |

On observe la formation importante de produits de déshydratation (3,7-diméthyl-2,6 octadiène et 3,7-diméthyl-6 octène) dans le cas du catalyseur M.

EXEMPLE 11

Un catalyseur monométallique à base de Ru est préparé selon le protocole décrit dans l'exemple 8, mais avec deux supports différents, charbon actif et alumine.

La fixation du plomb est réalisée dans les mêmes conditions que celles décrites dans l'exemple 1.

Deux catalyseurs ont ainsi été préparés, comparables au catalyseur K de l'exemple 9 (à support silice) :

| | Sn/Pb molaire | Sn/Pb poids |
|---|---|---|
| catalyseur N (sur charbon actif) 1,1 % Ru ; 4,4 % Pb | 1,95 | 4 |
| catalyseur P (sur alumine) 1 % Ru ; 4,1 % Pb | 2,0 | 4,10 |

Ils ont été testés dans les conditions de l'exemple 2.
Au bout de 8 heures de réaction, les résultats sont les suivants :

| Catalyseur | Conversion du citral (en poids) | Sélectivité % poids | | | | |
|---|---|---|---|---|---|---|
| | | 3,7 DMe 2,6octadiène | citronellol | citronellal | 3,7 DMe 6 octène | géraniol nérol |
| K | 98,8 | - | 5,5 | - | - | 94,5 |
| N | 88,0 | - | 7,5 | - | - | 92,5 |
| P | 97,0 | 9 | 3 | - | 2 | 86 |

On observe une formation importante des produits de déshydratation (3,7-diméthyl-2,6 octadiène et 3,7-diméthyl-6 octène) dans le cas du catalyseur P.

## Revendications

1. Procédé de production d'au moins un alcool insaturé dioléfinique par hydrogénation sélective du citral caractérisé en ce que l'on opère en présence d'au moins un catalyseur sur support à base de silice renfermant notamment d'une part 0,1 à 10 % en poids d'au moins un métal du groupe VIII choisi dans le groupe constitué par l'iridium, le platine, le rhodium et le ruthénium et d'autre part 0,01 à 10 % (exprimé en métal) en poids d'au moins un élément additionnel à base d'un métal du groupe IV.A choisi dans le groupe constitué par l'étain, le germanium et le plomb.

2. Procédé selon la revendication 1 dans lequel le catalyseur renferme en poids 0,5 à 5 % d'au moins un métal du groupe VIII et 0,1 à 5 % (exprimé en métal) d'au moins un élément additionnel à base d'un métal du groupe IV.

3. Procédé selon l'une des revendications 1 et 2 dans lequel le rapport molaire du métal du groupe IV sur

métal du groupe VIII est compris entre 0,8 et 3.

4. Procédé selon la revendication 3 dans lequel ledit rapport est compris entre 0,9 et 2,6.

5. Procédé selon l'une des revendications 1 à 4 dans lequel la pression est supérieure à 10 bars (1 mégapascal) et la température entre 0 et 100°C.

6. Procédé selon la revendication 5 dans lequel la pression est comprise entre 10 et 100 bars (1 et 10 mégapascals).

7. Procédé selon la revendication 6 dans lequel la pression est comprise entre 20 et 80 bars (2 et 8 mégapascals) et la température entre 30 et 80°C.

8. Procédé selon l'une des revendications 1 à 7 dans lequel le métal du groupe VIII est le rhodium.

9. Procédé selon l'une des revendications 1 à 7 dans lequel le métal du groupe VIII est le ruthénium.

10. Procédé selon l'une des revendications 1 à 9 appliqué à la fabrication d'au moins un alcool choisi dans le groupe constitué par le géraniol et le nérol.

## Claims

1. A method of producing at least one unsaturated diolefinic alcohol by selective hydrogenation of citral, characterised in that the operation takes place in the presence of at least one catalyst on a silica based carrier, containing in particular, firstly, 0.1 to 10% by weight of at least one group VIII metal selected from the group comprising iridium, platinum, rhodium and ruthenium and, secondly, 0.01 to 10% (expressed as metal) by weight of at least one additional element based on a group IV.A metal, selected from the group comprising tin, germanium and lead.

2. The method of Claim 1, wherein the catalyst contains (by weight) 0.5 to 5% of at least one group VIII metal and 0.1 to 5% (expressed as metal) of at least one additional element based on a group IV metal.

3. The method of Claim 1 or 2, wherein the molar ratio of the group IV metal to the group VIII metal is from 0.8:1 to 3:1.

4. The method of Claim 3, wherein said ratio is from 0.9:1 to 2.6:1.

5. The method of any of Claims 1 to 4, wherein the pressure is over 10 bars (1 Megapascal) and the temperature is from 0 to 100°C.

6. The method of Claim 5, wherein the pressure is from 10 to 100 bars (1 to 10 Megapascals).

7. The method of Claim 6, wherein the pressure is from 20 to 80 bars (2 to 8 Megapascals) and the temperature from 30 to 80°C.

8. The method of any of Claims 1 to 7, wherein the group VIII metal is rhodium.

9. The method of any of Claims I to 7, wherein the group VIII metal is ruthenium.

10. The method of any of Claims 1 to 9, applied to the preparation of at least one alcohol selected from the group comprising geraniol and nerol.

## Patentansprüche

1. Verfahren zur Herstellung wenigstens eines ungesättigten diolefinischen Alkohols durch selektive Hydrierung von Citral, dadurch gekennzeichnet, daß man in Gegenwart wenigstens eines Katalysators auf einem Träger auf Basis von Siliciumdioxid arbeitet, welcher insbesondere zum einen Teil 0,1 bis 10 Gew.-% wenigstens eines Metalls der Gruppe VIII, ausgewählt aus der Gruppe bestehend aus Iridium, Platin, Rhodium und Ruthenium, umfaßt und zum anderen Teil 0,01 bis 10 Gew.-% (ausgedrückt in Metall) wenigstens eines zusätzlichen Elementes auf Basis eines Metalls der Gruppe IV A, ausgewählt aus der Gruppe bestehend aus Zinn, Germanium und Blei.

**2.** Verfahren gemäß Anspruch 1, in welchem der Katalysator 0,5 bis 5 Gew.-% wenigstens eines Metalls der Gruppe VIII und 0,1 bis 5% (ausgedrückt in Metall) wenigstens eines zusätzlichen Elementes auf Basis eines Metalls der Gruppe IV umfaßt.

**3.** Verfahren gemäß einem der Anspruche 1 und 2, in welchem das molare Verhältnis von Metall der Gruppe IV zu Metall der Gruppe VIII zwischen 0,8 und 3 liegt.

**4.** Verfahren gemäß Anspruch 3, in welchem das Verhältnis zwischen 0,9 und 2,6 liegt.

**5.** Verfahren gemäß einem der Ansprüche 1 bis 4, in welchem der Druck über 10 bar (1 MPa) und die Temperatur zwischen 0 und 100°C liegt.

**6.** Verfahren gemäß Anspruch 5, in welchem der Druck zwischen 10 und 100 bar (1 und 10 MPa) liegt.

**7.** Verfahren gemäß Anspruch 6, in welchem der Druck zwischen 20 und 80 bar (2 und 8 MPa) und die Temperatur zwischen 30 und 80°C liegt.

**8.** Verfahren gemäß einem der Ansprüche 1 bis 7, in welchem das Metall der Gruppe VIII Rhodium ist.

**9.** Verfahren gemäß einem der Ansprüche 1 bis 7, in welchem das Metall der Gruppe VIII Ruthenium ist.

**10.** Verfahren gemäß einem der Ansprüche 1 bis 9, angewendet auf die Herstellung wenigstens eines Alkoholes, ausgewählt aus der Gruppe bestehend aus Geraniol und Nerol.